# EUROPEAN PATENT SPECIFICATION

(11) **EP 0 366 329 B1**
(45) Date of publication and mention of the grant of the patent: **20.03.1996**
(21) Application number: 89310625.2
(22) Date of filing: 17.10.1989
(51) Int. Cl.: C07D 231/16, C07D 231/12, C07D 401/04

(54) **Halogeno-4-methylpyrazoles and process for preparing the same**
Halogeno-4-Methylpyrazole und Verfahren zu ihrer Herstellung
4-méthylpyrazoles halogénés et procédé pour les préparer

(30) Priority: 27.10.1988 JP 271438/88
(43) Date of publication of application: 02.05.1990
(73) Proprietor: NISSAN CHEMICAL INDUSTRIES, LIMITED, Chiyoda-ku Tokyo 101 (JP)
(72) Inventor: Iwasawa, Yoshihiro c/o Central Research Institute, Funabashi-shi Chiba-ken (JP); Yamamoto, Susumu c/o Central Research Institute, Funabashi-shi Chiba-ken (JP); Suzuki, Kenji c/o Central Research Institute, Funabashi-shi Chiba-ken (JP); Murakami, Hiroshi c/o Central Research Institute, Funabashi-shi Chiba-ken (JP); Suzuki, Fumio c/o Central Research Institute, Funabashi-shi Chiba-ken (JP)
(74) Representative: Watkins, David

(56) References cited:
- EP-A- 0 191 282
- EP-A- 0 202 169
- EP-A- 0 257 479
- EP-A- 0 350 176
- CHEMICAL ABSTRACTS, vol. 66, no. 21, May 22, 1967, page 8889, abstract no. 94950x, Columbus, Ohio, US; JOSE ELGUERO et al.: "Azoles. Bromination of pyrazolecarboxylic acids and esters", column 1; & C.R. Acad. Sci. Paris, Ser. C 263 (23), 1456-9 (1966)
- CHEMICAL ABSTRACTS, vol. 71, no. 23, December 8, 1969, page 372, abstract no. 112859j, Columbus, Ohio, US; PH. BOUCHET et al.: "Azoles. Halogenation of 4-substituted pyrazoles", column 2; & C.R. Acad. Sci. Ser. C 1969, 269, no.10, pages 570-572
- JOURNAL OF AGRICULTURAL AND FOOD CHEMISTRY, vol. 25, no. 4, pages 884-888, 1977, GABRIEL KORNIS et al.: "Herbicidal Activity of Alkylpyrazole Amides"

## Description

This invention relates to halogeno-4-methylpyrazoles useful as intermediates for pharmaceuticals and agricultural chemicals, etc. and processes for producing the same, more particularly to 5-halogeno-1-substituted-4-methylpyrazoles and 3,5-dihalogeno-1-substituted-4-methylpyrazoles and processes for producing these.

Journal of Agricultural Food Chemistry (J. Agrc. Food. Chem.), vol. 25, No. 4, p. 884 (1977) discloses a process for preparing 4-methyl-3,5-dibromopyrazole which comprises permitting butyl lithium to act on 3,4,5-tribromopyrazole, followed by substitution with methyl iodide.

However, this process cannot be said to be an industrial production process, because synthesis of 3,4,5-tribromopyrazole is complicated, and moreover the synthesis steps are lengthy, and yet expensive butyl lithium is used.

Chemical Abstract Vol. 66, 94950x discloses a process for preparing 3-bromo-1,4-dimethylpyrazole which comprises brominating 1,4-dimethyl-5-hydroxycarbonylpyrazole, followed by decarboxylation.

However, this process cannot be said to be an industrial production process, because 1,4-dimethyl-5-carboxypyrazole is expensive, and the synthesis steps are complicated.

Bouchet et al. C.R. Acad. Sc. Paris, Ser. C, 1969, 269(10), 570-572 indicate that the bromination of 3,4,5-trimethyl pyrazole with bromine or N-bromosuccinimide results in the formation of the unstable 4-bromo-3,4,5-trimethyl pyrazole. Bromination in the presence of methanol forms 5-methyl-3,4,5-trimethyl pyrazolenine. Bromination of 3,5-dimethyl-4-ethyl pyrazole and 3,5-diethyl-4-methyl pyrazole results in corresponding 4-bromoisopyrazoles.

Bromination of 4-methylpyrazole results in the formation of the symmetrical trimer.

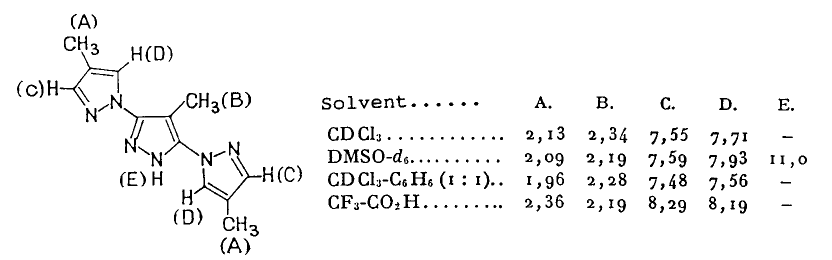

On the other hand, iodination of 4-methyl pyrazole results in the production of 3(5)-iodo-4-methylpyrazole.

EP-A-0202169 discloses certain N-phenyl substituted pyrazoles and describes the synthesis of various compounds with halogen substituents in the pyrazole ring by direct halogenation processes. No information is given as to the selectivity or the extent to which the presence of other substituents in the pyrazole ring determines the orientation of the products of the selectivity of the reaction.

The present inventors have investigated intensively about processes for obtaining halogeno-4-methylpyrazoles with good yield, and consequently accomplished the present invention.

More specifically, the present invention concerns halogeno-4-methylpyrazoles represented by the formula (I): 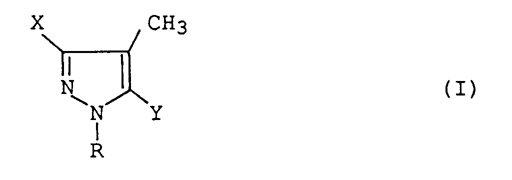
(wherein R represents an alkyl group having 1 to 4 carbon atoms, a substituted or unsubstituted phenyl group (as defined below) or a substituted or unsubstituted pyridyl group (as defined below), X represents a hydrogen atom or a halogen atom and Y represents a halogen atom) but with the proviso that 3,5-dichloro-1,4-dimethylpyrazole is excluded,
and a process for preparing halogeno-4-methylpyrazoles represented by the formula (I), which comprises reacting 1-substituted-4-methylpyrazoles of the formula (II): 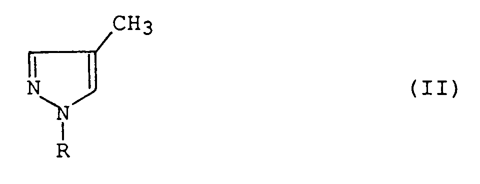
(wherein R has the meaning given above)
with chlorine or bromine.

In the halogeno-4-methylpyrazoles of the above formula (I) and the 1-substituted-4-methylpyrazoles of the formula (II), the alkyl group of R having 1 to 4 carbon atoms may include a methyl group, an ethyl group, a n-propyl group, an i-propyl group, a n-butyl group, an i-butyl group, or a t-butyl group.

The substituted or unsubstituted phenyl group is a phenyl group, a 2-methylphenyl group, a 3-methylphenyl group, a 4-methylphenyl group, a 2-ethylphenyl group, a 3-ethylphenyl group, a 4-ethylphenyl group, a 2-n-propylphenyl group, a 2-i-propylphenyl group, a 3-n-propylphenyl group, a 3-i-propylphenyl group, a 4-n-propylphenyl group, a 4-i-propylphenyl group, a 2-t-butylphenyl group, a 4-n-butylphenyl group, a 4-i-butylphenyl group, a 4-t-butylphenyl group, a 2,4-dimethylphenyl group, a 2,6-dimethylphenyl group, a 2,4-diethylphenyl group, a 2,6-diethylphenyl group, a 2-methoxyphenyl group, a 3-methoxyphenyl group, a 4-methoxyphenyl group, a 2-ethoxyphenyl group, a 3-ethoxyphenyl group, a 4-ethoxyphenyl group, a 2-n-propoxyphenyl group, a 2-i-propoxyphenyl group, a 3-n-propoxyphenyl group, a 4-n-propoxyphenyl group, a 4-i-propoxyphenyl group, a 2-n-butoxyphenyl group, a 4-n-butoxyphenyl group, a 4-i-butoxyphenyl group, a 4-t-butoxyphenyl group, a 2-chlorophenyl group, a 3-chlorophenyl group, a 4-chlorophenyl group, a 2-fluorophenyl group, a 3-fluorophenyl group, a 4-fluorophenyl group, a 2,4-dichlorophenyl group, a 2,4-difluorophenyl group, a 2,6-dichlorophenyl group, a 2,6-difluorophenyl group, a 2-nitrophenyl group, a 3-nitrophenyl group, a 4-nitrophenyl group, a 2-difluoromethoxyphenyl group, a 3-difluoromethoxyphenyl group, or a 4-difluoromethoxyphenyl group. The substituted or unsubstituted pyridyl group is a 2-pyridyl group, a 3-methyl-2-pyridyl group, a 4-methyl-2-pyridyl group, a 5-methyl-2-pyridyl group, a 6-methyl-2-pyridyl group, a 3-chloro-2-pyridyl group, a 4-chloro-2-pyridyl group, a 5-chloro-2-pyridyl group, a 6-chloro-2-pyridyl group, a 3,5-dichloro-2-pyridyl group, a 3-trifluoromethyl-2-pyridyl group, a 5-trifluoromethyl-2-pyridyl group, a 3-pyridyl group, a 2-methyl-3-pyridyl group, a 4-methyl-3-pyridyl group, a 5-methyl-3-pyridyl group, a 6-methyl-3-pyridyl group, a 2-chloro-3-pyridyl group, a 5-chloro-3-pyridyl group, a 6-chloro-3-pyridyl group, a 4-pyridyl group, a 2-methyl-4-pyridyl group, a 3-methyl-4-pyridyl group, a 2-chloro-4-pyridyl group, or a 3-chloro-4-pyridyl group.

Specific examples of the halogeno-4-methylpyrazoles of the formula (I) may include 5-chloro-1,4-dimethylpyrazole, 5-bromo-1,4-dimethylpyrazole, 5-chloro-1-phenyl-4-methylpyrazole, 5-bromo-1-phenyl-4-methylpyrazole, 5-chloro-1-(2-pyridyl)-4-methylpyrazole, 5-chloro-1-(3-pyridyl)-4-methylpyrazole, 5-chloro-1-(4-pyridyl)-4-methylpyrazole, 3,5-dibromo-1,4-dimethylpyrazole, 3,5-dichloro-1-phenyl-4-methylpyrazole, 3,5-dibromo-1-phenyl-4-methylpyrazole, 3,5-dichloro-1-(2-pyridyl)-4-methylpyrazole, 3,5-dichloro-1-(3-pyridyl)-4-methylpyrazole, 3,5-dichloro-1-(4-pyridyl)-4-methylpyrazole, 3,5-dibromo-1-(2-pyridyl)-4-methylpyrazole, 3,5-dibromo-1-(3-pyridyl)-4-methylpyrazole, 3,5-dibromo-1-(4-pyridyl)-4-methylpyrazole, and the like.

Specific examples of the 1-substituted-4-methylpyrazoles of the formula (II) may include 1,4-dimethylpyrazole, 1-ethyl-4-methylpyrazole, 1-n-propyl-4-methylpyrazole, 1-i-propyl-4-methylpyrazole, 1-n-butyl-4-methylpyrazole, 1-i-butyl-4-methylpyrazole, 1-t-butyl-4-methylpyrazole, 1-phenyl-4-methylpyrazole, 1-(2-pyridyl)-4-methylpyrazole, 1-(3-pyridyl)-4-methylpyrazole, 1-(4-pyridyl)-4-methylpyrazole and the like (In the above, n means normal, i means iso and t means tertiary, respectively).

The 1-substituted-4-methylpyrazoles represented by the formula (II) can be produced with good yield by the reaction between 2,3-dichloro-2-methylpropanal and a substituted hydrazine (see Japanese Provisional Patent Publication No. 270345/1988).

In the halogeno-4-methylpyrazoles of the above formula (I), as the halogen atom of X and Y, a chlorine atom and a bromine atom may be employed.

In the following, the reaction conditions of the 1-substituted-4-methylpyrazoles of the formula (II) and halogen are to be described in detail.

As the reaction temperature, a range generally of -10 to 100 °C is employed.

As the reaction time, a range generally of 5 minutes to 15 hours is employed.

The present invention can be practiced with no solvent, but a solvent can be also used.

As the solvent, there may be included halogenated aliphatic hydrocarbons such as methylene chloride, chloroform, carbon tetrachloride, dichloroethane, tetrachloroethane, etc., halo-substituted aromatic hydrocarbons such as chlorobenzene, dichlorobenzene, etc.

The above solvents can be also used as a mixture of two or more kinds.

As the solvent, one or more solvents selected from halogenated aliphatic hydrocarbon solvents such as dichloroethane, etc., halo-substituted aromatic hydrocarbons such as o-dichlorobenzene, etc. are particularly preferred.

An amount of the solvent used may be generally in the range of 0.1 to 20 parts by weight, preferably 1 to 10 parts by weight, based on 1 part by weight of the 1-substituted-4-methylpyrazoles of the formula (II). Also, the solvent may be used those which are commercially available, but if necessary, when the solvent is subjected to drying treatment with molecular sieves, etc., good results can be obtained.

As the halogen, chlorine and bromine may be mentioned. The halogen can be used either in liquid or gaseous state.

An amount of the halogen used may be generally in the range of 0.5 to 1.6 moles, preferably 0.8 to 1.33 moles, per one mole of the 1-substituted-4-methylpyrazoles of the formula (II), when producing 5-halogeno-1-substituted-4-methylpyrazoles which is monohalogeno-material among the compounds represented by the formula (I).

On the other hand, in the case of producing 3,5-dihalogeno-1-substituted-4-methylpyrazoles which is dihalogeno-material among the compounds represented by the formula (I), an amount of the halogen may be generally in the range of 1.4 to 3.0 moles, preferably 1.8 to 2.5 moles, based on one mole of the 1-substituted-4-methylpyrazoles of the formula (II).

As the reaction method between the 1-substituted-4-methylpyrazoles represented by the formula (II) and halogen, there may be employed the reaction in which the reaction is carried out by blowing with gaseous state or adding dropwise a halogen into 1-substituted-4-methylpyrazoles represented by the formula (II), or the method in which the 1-substituted-4-methylpyrazoles represented by the formula (II) are added dropwise into halogen, etc.

After completion of the reaction, the halogeno-4-methylpyrazoles represented by the formula (I) can be isolated by direct distillation, etc., but if necessary, it can be also obtained with good results by removing almost all the parts of remaining halogen and by-produced hydrohalogenic acid in the reaction mixture by such operation as reducing pressure or blowing of nitrogen as a pretreatment, subsequently treatment with an alkaline aqueous solution such as sodium hydroxide, potassium hydroxide, etc. completely, and then purification such as distillation, etc.

As the halogenating agent, in addition to the above chlorine and bromine atoms, there may be also used, for example, sulfuryl chloride, sulfuryl bromide, N-chlorosuccinimide, N-bromosuccinimide, tert-butyl hypochloride, etc.

Also, the reaction can be carried out without using a deacidificating agent, but an organic base such as triethylamine, N,N-dimethylaniline, pyridine, etc. or an inorganic base such as sodium carbonate, potassium carbonate, sodium hydroxide, etc. may be used.

The reaction can be carried out without using a catalyst, but as the catalyst, a Lewis acid such as BF₃, AlCl₃, FeCl₃, SbCl₃, SbCl₅, etc., a metal halide such as CuCl, CuCl₂, etc., a metal such as iron, copper, etc., or a halogen such as iodine, etc. may be added thereto.

By the reaction of the 1-substituted-4-methylpyrazoles represented by the formula (II) and halogen, halogeno-4-methylpyrazoles represented by the formula (I) can be obtained easily at high yield.

The 5-halogeno-1-substituted-4-methylpyrazoles which are monohalogeno-material among halogeno-4-methylpyrazoles represented by the formula (I) can be oxidized at the methyl group at the 4-position, followed by esterification to produce 5-halogeno-1-alkyl-4-alkoxycarbonylpyrazoles which are useful as intermediate products in the preparation of herbicides (Japanese Provisional Patent Publication No. 122488/1984, corresponding to EP-A-0087780).

Also, the 3,5-dihalogeno-1-substituted-4-methylpyrazoles which are dihalogeno material among halogeno-4-methyl-pyrazoles represented by the formula (I) can be oxidized at the methyl group at the 4-position, followed by esterification to produce 3,5-dihalogeno-4-alkoxycarbonylpyrazoles which are useful as intermediate products in the preparation of herbicides (Japanese provisional Patent publication No. 208977/1985, corresponding to US-A-4668277).

The present invention is described in detail below by referring to Examples, by which the present invention is not limited at all.

### Example 1

Into a mixture of 19.3 g (0.2 mole) of 1,4-dimethylpyrazole and 96 g of 1,2-dichloroethane was blown 18.9 g (0.266 mole) of chlorine under stirring for one hour, while maintaining the reaction temperature at 25 to 35 °C.

After completion of blowing, the temperature was returned to room temperature, and analyzed by liquid chromatography to find that 17.5 g of 5-chloro-1,4-dimethylpyrazole was contained.

The yield of 5-chloro-1,4-dimethylpyrazole was 67 %.

The above reaction mixture was washed with 30 g of a 20 % aqueous sodium hydroxide solution, and after evaporation of the solvent, distillation was carried out by use of Widmer to give 17 g of 5-chloro-1,4-dimethylpyrazole of a purity of 95 % at a boiling point range of 100 to 110 °C/120 mm Hg (16 kPa).
¹H-NMR (CDCl₃):
δ 1.97 (3H, s), 3.75 (3H, s), 7.3 (1H, s)

### Example 2

Into a mixture of 9.65 g (0.1 mole) of 1,4-dimethylpyrazole and 48 g of 1,2-dichloroethane was blown 15.6 g (0.22 mole) of chlorine under stirring for one hour, while maintaining the reaction temperature at 5 to 15 °C.

After completion of blowing, the temperature was returned to room temperature, and analyzed by liquid chromatography to find that 14.4 g of 3,5-dichloro-1,4-dimethylpyrazole was contained.

The yield of 3,5-dichloro-1,4-dimethylpyrazole was 87 %.

After evaporation of the solvent from the above reaction mixture, distillation was carried out to give 15.2 g of 3,5-dichloro-1,4-dimethylpyrazole of a purity of 90 % at a boiling point range of 98 to 109 °C/120 mmHg (16 kPa).
¹H-NMR (CDCl₃):
δ 1.9 (3H, s), 3.7 (3H, s)
3,5-dichloro-1,4-dimethylpyrazole is not claimed in this Application.

### Example 3

Into a mixture of 3.1 g (0.0323 mole) of 1,4-dimethylpyrazole and 7 g of carbon tetrachloride was blown 7 g (0.097 mole) of chlorine under stirring for one hour, while maintaining the reaction temperature at 80 °C.

After completion of blowing, the reaction temperature was returned to room temperature, and after evaporation of the solvent, distillation was carried out to give 3.6 g of 3,5-dichloro-1,4-dimethylpyrazole of a purity of 95 % at a boiling point range of 98 to 109 °C/20 mmHg (3 kPa).

The yield of 3,5-dichloro-1,4-dimethylpyrazole was found to be 64 %.

3,5-dichloro-1,4-dimethylpyrazole is not claimed in this Application.

### Example 4

Into a mixture of 9.65 g (0.1 mole) of 1,4-dimethylpyrazole, 0.5 g of iron powder and 60 g of 1,2-dichloroethane was added dropwise 48 g (0.3 mole) of bromine under stirring for one hour, while maintaining the reaction temperature at 10 to 35 °C, and stirring was continued at 80 °C for 15 hours.

After completion of the reaction, bromine was removed under reduced pressure and the reaction mixture was neutralized with a 10 % sodium hydroxide. Subsequently, insolubles were removed by filtration, washed with water and after evaporation of the solvent, distillation was carried out to give 9.3 g of 3,5-dibromo-1,4-dimethylpyrazole of a purity of 98 % at a boiling point range of 124 to 128 °C/20 mmHg (3 kPa).

The yield of 3,5-dibromo-1,4-dimethylpyrazole was found to be 36 %.
¹H-NMR (CDCl₃):
δ 1.95 (3H, s), 3.8 (3H, s)

### Example 5

Into a mixture of 2.5 g (0.0164 mole) of 4-methyl-1-phenylpyrazole and 20 g of 1,2-dichloroethane was blown 1.0 g (0.0141 mole) of chlorine under stirring for 15 minutes, while maintaining the reaction temperature at 20 to 25 °C.

The reaction mixture was analyzed by gas chromatography and 1.3 g of 5-chloro-4-methyl-1-phenylpyrazole was found to be contained. The yield was 41 %.
GC-MS:
M/e: 192 (M⁺), 157 (M⁺ - Cl), 130, 89, 77.

Into the above reaction mixture was further blown 3.0 g (0.0423 mole) of chlorine under stirring for 45 minutes, while maintaining the reaction temperature at 30 to 35 °C. After evaporation of the solvent, the residue was recrystallized with n-hexane to give 1.1 g of 3,5-dichloro-1-(4-chlorophenyl)-4-methylpyrazole having a melting point of 90 to 91.5 °C.

The isolated yield of 3,5-dichloro-1-(4-chlorophenyl)-4-methylpyrazole was 26 %.
¹H-NMR (CDCl₃):
δ 2.09 (3H, s), 7.56 (4H, s)

## Claims

1. Halogeno-4-methylpyrazoles represented by the Formula (I): 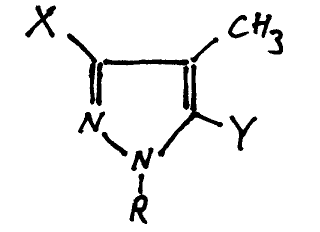 wherein R represents an alkyl group having 1 to 4 carbon atoms, phenyl, 2-methylphenyl, 3-methylphenyl, 4-methylphenyl, 2-ethylphenyl, 3-ethylphenyl, 4-ethylphenyl, 2-n-propylphenyl, 2-i-propylphenyl, 3-n-propylphenyl, 3-i-propylphenyl, 4-n-propylphenyl, 4-i-propylphenyl, 2-t-butylphenyl, 4-n-butylphenyl, 4-i-butylphenyl, 4-t-butylphenyl, 2,4-diethylphenyl, 2,6-diethylphenyl, 2,4-dimethylphenyl, 2,6-dimethylphenyl, 2-methoxyphenyl, 3-methoxyphenyl, 4-methoxyphenyl, 2-ethoxyphenyl, 3-ethoxyphenyl, 4-ethoxyphenyl, 2-n-propoxyphenyl, 2-i-propoxyphenyl, 3-n-propoxyphenyl, 4-n-propoxyphenyl, 4-i-propoxyphenyl, 2-n-butoxyphenyl, 4-n-butoxyphenyl, 4-i-butoxyphenyl, 4-t-butoxyphenyl, 2-chlorophenyl, 3-chlorophenyl, 4-chlorophenyl, 2-fluorophenyl, 3-fluorophenyl, 4-fluorophenyl, 2,4-dichlorophenyl, 2,4-difluorophenyl, 2,6-dichlorophenyl, 2,6-difluorophenyl, 2-nitrophenyl, 3-nitrophenyl, 4-nitrophenyl, 2-difluoromethoxyphenyl, 3-difluoromethoxyphenyl, 4-difluoromethoxyphenyl, 2-pyridyl, 3-methyl-2-pyridyl, 4-methyl-2-pyridyl, 5-methyl-2-pyridyl, 6-methyl-2-pyridyl, 3-chloro-2-pyridyl, 4-chloro-2-pyridyl, 5-chloro-2-pyridyl, 6-chloro-2-pyridyl, 3,5-dichloro-2-pyridyl, 3-trifluoromethyl-2-pyridyl, 5-trifluoromethyl-2-pyridyl, 3-pyridyl, 2-methyl-3-pyridyl, 4-methyl-3-pyridyl, 5-methyl-3-pyridyl, 6-methyl-3-pyridyl, 2-chloro-3-pyridyl, 5-chloro-3-pyridyl, 6-chloro-3-pyridyl, 4-pyridyl, 2-methyl-4-pyridyl, 3-methyl-4-pyridyl, 2-chloro-4-pyridyl, or 3-chloro-4-pyridyl; X represents a hydrogen atom, chlorine or bromine; and Y represents chlorine or bromine, with the proviso that 3,5-dichloro-1,4-dimethyl-pyrazole is excluded.

2. Halogeno-4-methylpyrazoles according to Claim 1, wherein R is an alkyl group having 1 to 4 carbon atoms, a phenyl group or a pyridyl group; X is a hydrogen atom, a chlorine atom or a bromine atom; and Y is a chlorine atom or a bromine atom, with the proviso that 3,5-dichloro-1,4-dimethylpyrazole is excluded.

3. Halogeno-4-methylpyrazoles according to Claim 1, wherein R is an alkyl group having 1 to 4 carbon atoms; X is a hydrogen atom, a chlorine atom or a bromine atom; and Y is a chlorine atom or a bromine atom, with the proviso that 3,5-dichloro-1,4-dimethylpyrazole is excluded.

4. Halogeno-4-methylpyrazoles according to Claim 1, wherein R is a methyl group; X is a hydrogen atom; and Y is a chlorine atom.

5. A process for preparing halogeno-4-methylpyrazoles according to Claim 1 which comprises reacting 1-substituted-4-methylpyrazoles of the formula (II): 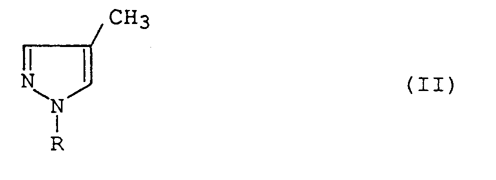 wherein R has the meaning given in Claim 1, with chlorine or bromine.

6. A process
according to Claim 5, wherein R is an alkyl group having 1 to 4 carbon atoms, a phenyl group or a pyridyl group; X is a hydrogen atom, a chlorine atom or a bromine atom; and Y is a chlorine atom or a bromine atom.

7. A process
according to Claim 5, wherein R is an alkyl group having 1 to 4 carbon atoms; X is a hydrogen atom, a chlorine atom or a bromine atom; Y is a chlorine atom or a bromine atom.

8. A process
according to Claim 5, wherein R is an alkyl group having 1 to 4 carbon atoms; X is a hydrogen atom or a chlorine atom; and Y is a chlorine atom.

## Patentansprüche

1. Halogen-4-Methylpyrazole, dargestellt durch die Formel (I): 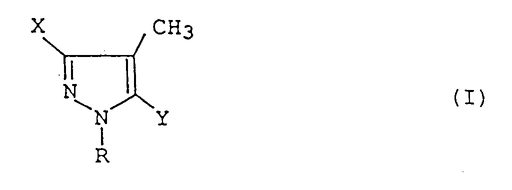 worin R eine Alkylgruppe mit 1 bis 4 Kohlenstoffatomen darstellt, sowie Phenyl, 2-Methylphenyl, 3-Methylphenyl, 4-Methylphenyl, 2-Ethylphenyl, 3-Ethylphenyl, 4-Ethylphenyl, 2-n-Propylphenyl, 2-i-Propylphenyl, 3-n-Propylphenyl, 3-i-Propylphenyl, 4-n-Propylphenyl, 4-i-Propylphenyl, 2-t-Butylphenyl, 4-n-Butylphenyl, 4-i-Butylphenyl, 4-t-Butylphenyl, 2,4-Diethylphenyl, 2,6-Diethylphenyl, 2,4-Dimethylphenyl, 2,6-Dimethylphenyl, 2-Methoxyphenyl, 3-Methoxyphenyl, 4-Methoxyphenyl, 2-Ethoxyphenyl, 3-Ethoxyphenyl, 4-Ethoxyphenyl, 2-n-Propoxyphenyl, 2-i-Propoxyphenyl, 3-n-Propoxyphenyl, 4-n-Propoxyphenyl, 4-i-Propoxyphenyl, 2-n-Butoxyphenyl, 4-n-Butoxyphenyl, 4-i-Butoxyphenyl, 4-t-Butoxyphenxyl, 2-Chlorphenyl, 3-Chlorphenyl, 4-Chlorphenyl, 2-Fluorphenyl, 3-Fluorphenyl, 4-Fluorphenyl, 2,4-Dichlorphenyl, 2,4-Difluorphenyl, 2,6-Dichlorphenyl, 2,6-Difluorphenyl, 2-Nitrophenyl, 3-Nitrophenyl, 4-Nitrophenyl, 2-Difluormethoxyphenyl, 3-Difluormethoxyphenyl, 4-Difluormethoxyphenyl, 2-Pyridyl, 3-Methyl-2-pyridyl, 4-Methyl-2-pyridyl, 5-Methyl-2-pyridyl, 6-Methyl-2-pyridyl, 3-Chlor-2-pyridyl, 4-Chlor-2-pyridyl, 5-Chlor-2-pyridyl, 6-Chlor-2-pyridyl, 3,5-Dichlor-2-pyridyl, 3-Trifluormethyl-2-pyridyi, 5-Trifluormethyl-2-pyridyl, 3-Pyridyl, 2-Methyl-3-pyridyl, 4-Methyl-3-pyridyl, 5-Methyl-3-pyridyl, 6-Methyl-3-pyridyl, 2-Chlor-3-pyridyl, 5-Chlor-3-pyridyl, 6-Chlor-3-pyridyl, 4-Pyridyl, 2-Methyl-4-pyridyl, 3-Methyl-4-pyridyl, 2-Chlor-4-pyridyl, oder 3-Chlor-4-pyridyl; X ein Wasserstoffatom, Chlor oder Brom darstellt; und Y Chlor oder Brom darstellt, mit der Bedingung, daß 3,5-Dichlor-1,4-dimethyl-pyrazol ausgeschlossen ist.

2. Halogen-4-Methylpyrazole nach Anspruch 1, bei welchen R eine Alkylgruppe mit 1 bis 4 Kohlenstoffatomen, eine Phenylgruppe oder eine Pyridylgruppe ist; X ein Wasserstoffatom, ein Chloratom oder ein Bromatom ist; und Y ein Chloratom oder ein Bromatom ist, mit der Bedingung, daß 3,5 Dichlor-1,4-dimethyl-pyrazol ausgeschlossen ist.

3. Halogen-4-Methylpyrazole nach Anspruch 1, bei welchen R eine Alkylgruppe mit 1 bis 4 Kohlenstoffatomen ist; X ein Wasserstoffatom, ein Chloratom oder ein Bromatom ist; und Y ein Chloratom oder ein Bromatom ist, mit der Bedingung, daß 3,5 Dichlor-1,4-dimethyl-pyrazol ausgeschlossen ist.

4. Halogen-4-Methylpyrazole nach Anspruch 1, bei welchen R eine Methylgruppe ist; X ein Wasserstoffatom ist; und Y ein Chloratom ist.

5. Verfahren zur Herstellung von Halogen-4-Methylpyrazolen nach Anspruch 1, welches aus der Reaktion von 1-substituierten-4-Methylpyrazolen der Formel (II): 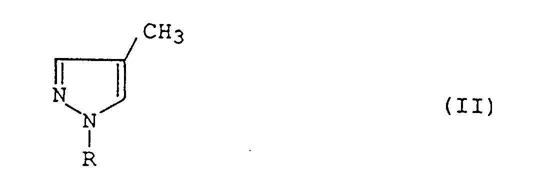 worin R die in Anspruch 1 angegebene Bedeutung hat,
mit Chlor oder Brom besteht.

6. Verfahren nach Anspruch 5, bei welchen R eine Alkylgruppe mit 1 bis 4 Kohlenstoffatomen, eine Phenylgruppe oder eine Pyridylgruppe ist; X ein Wasserstoffatom, ein Chloratom oder ein Bromatom ist; und Y ein Chloratom oder ein Bromatom ist.

7. Verfahren nach Anspruch 5, bei welchen R eine Alkylgruppe mit 1 bis 4 Kohlenstoffatomen ist; X ein Wasserstoffatom, ein Chloratom oder ein Bromatom ist; und Y ein Chloratom oder ein Bromatom ist.

8. Verfahren nach Anspruch 5, bei welchen R eine Alkylgruppe mit 1 bis 4 Kohlenstoffatomen ist; X ein Wasserstoffatom oder ein Chloratom ist; und Y ein Chloratom ist.

## Revendications

1. Halogéno-4-méthylpyrazoles, représentés par la formule (I): 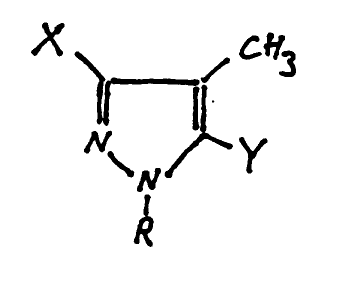 dans laquelle R représente un groupe alkyle comportant 1 à 4 atomes de carbone, un groupe phényle, 2-méthylphényle, 3-méthylphényle, 4-méthylphényle, 2-éthylphényle, 3-éthylphényle, 4-éthylphényle, 2-n-propylphényle, 2-i-propylphényle, 3-n-propylphényle, 3-i-propylphényle, 4-n-propylphényle, 4-i-propylphényle, 2-t-butylphényle, 4-n-butylphényle, 4-i-butylphényle, 4-t-butylphényle, 2,4-diéthylphényle, 2,6-diéthylphényle, 2,4-diméthylphényle, 2,6-diméthylphényle, 2-méthoxyphényle, 3-méthoxyphényle, 4-méthoxyphényle, 2-éthoxyphényle, 3-éthoxyphényle, 4-éthoxyphényle, 2-n-propoxyphényle, 2-i-propoxyphényle, 3-n-propoxyphényle, 4-n-propoxyphényle, 4-i-propoxyphényle, 2-n-butoxyphényle, 4-n-butoxyphényle, 4-i-butoxyphényle, 4-t-butoxyphényle, 2-chlorophényle, 3-chlorophényle, 4-chlorophényle, 2-fluorophényle, 3-fluorophényle, 4-fluorophényle, 2,4-dichlorophényle, 2,4-difluorophényle, 2,6-dichlorophényle, 2,6-difluorophényle, 2-nitrophényle, 3-nitrophényle, 4-nitrophényle, 2-difluorométhoxyphényle, 3-difluorométhoxyphényle, 4-difluorométhoxyphényle, 2-pyridyle, 3-méthyl-2-pyridyle, 4-méthyl-2-pyridyle, 5-méthyl-2-pyridyle, 6-méthyl-2-pyridyle, 3-chloro-2-pyridyle, 4-chloro-2-pyridyle, 5-chloro-2-pyridyle, 6-chloro-2-pyridyle, 3,5-dichloro-2-pyridyle, 3-trifluorométhyl-2-pyridyle, 5-trifluorométhyl-2-pyridyle, 3-pyridyle, 2-méthyl-3-pyridyle, 4-méthyl-3-pyridyle, 5-méthyl-3-pyridyle, 6-méthyl-3-pyridyle, 2-chloro-3-pyridyle, 5-chloro-3-pyridyle, 6-chloro-3-pyridyle, 4-pyridyle, 2-méthyl-4-pyridyle, 3-méthyl-4-pyridyle, 2-chloro-4-pyridyle ou 3-chloro-4-pyridyle; X représente un atome d'hydrogène, de chlore ou de brome; et Y représente un atome de chlore ou de brome, à condition d'exclure le 3,5-dichloro-1,4-diméthylpyrazole.

2. Halogéno-4-méthylpyrazoles selon la revendication 1, dans lesquels R est un groupe alkyle comportant 1 à 4 atomes de carbone, un groupe phényle ou un groupe pyridyle; X est un atome d'hydrogène, un atome de chlore ou un atome de brome; et Y est un atome de chlore ou un atome de brome, à condition d'exclure le 3,5-dichloro-1,4-diméthylpyrazole.

3. Halogéno-4-méthylpyrazoles selon la revendication 1, dans lesquels R est un groupe alkyle comportant 1 à 4 atomes de carbone; X est un atome d'hydrogène, un atome de chlore ou un atome de brome; et Y est un atome de chlore ou un atome de brome, à condition d'exclure le 3,5-dichloro-1,4-diméthylpyrazole.

4. Halogéno-4-méthylpyrazoles selon la revendication 1, dans lesquels R est un groupe méthyle; X est un atome d'hydrogène; et Y est un atome de chlore.

5. Procédé de préparation d'halogéno-4-méthylpyrazoles selon la revendication 1, qui comprend la réaction de 4-méthylpyrazoles 1-substitués de formule (II): 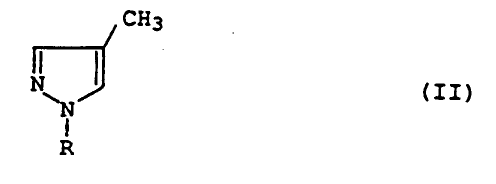 dans laquelle R a la signification donnée dans la revendication 1, avec du chlore ou du brome.

6. Procédé selon la revendication 5, dans lequel R est un groupe alkyle comportant 1 à 4 atomes de carbone, un groupe phényle ou un groupe pyridyle; X est un atome d'hydrogène, un atome de chlore ou un atome de brome; et Y est un atome de chlore ou un atome de brome.

7. Procédé selon la revendication 5, dans lequel R est un groupe alkyle comportant 1 à 4 atomes de carbone; X est un atome d'hydrogène, un atome de chlore ou un atome de brome; et Y est un atome de chlore ou un atome de brome.

8. Procédé selon la revendication 5, dans lequel R est un groupe alkyle comportant 1 à 4 atomes de carbone; X est un atome d'hydrogène ou un atome de chlore; et Y est un atome de chlore.
